(19)

Europäisches
Patentamt
European
Patent Office
Office européen
des brevets

(11) **EP 4 755 911 A2**

(12) **EUROPEAN PATENT APPLICATION**

(43) Date of publication:
**10.06.2026  Bulletin 2026/24**

(21) Application number: **25221368.1**

(22) Date of filing: **08.12.2025**

(51) International Patent Classification (IPC):
*C07K 16/26* $^{(2006.01)}$      *G01N 33/74* $^{(2006.01)}$

(52) Cooperative Patent Classification (CPC):
**C07K 16/26; G01N 33/74; C07K 2317/34**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority:   **06.12.2024   CN 202411798937**

(71) Applicant: **Shenzhen Mindray Bio-Medical
Electronics Co., Ltd.
Shenzhen, Guangdong 518057 (CN)**

(72) Inventors:
• **ZHANG, Haifeng
Shenzhen 518057 (CN)**

• **LIU, Kun
Shenzhen 518057 (CN)**
• **CHEN, Puguang
Shenzhen 518057 (CN)**
• **LI, Ke
Shenzhen 518057 (CN)**

(74) Representative: **KIPA AB
Drottninggatan 11
252 21 Helsingborg (SE)**

Remarks:
The complete document including Reference
Table(s) and the Sequence Listing(s) can be
downloaded from the EPO website

(54) **ANTIBODY PAIR FOR BINDING TO MATURE PROLACTIN**

(57)    The present disclosure provides an antibody pair capable of binding to human mature prolactin, which comprises: a first monoclonal antibody or antigen-binding fragment thereof capable of binding to the N-terminal region of human mature prolactin, and a second monoclonal antibody or antigen-binding fragment thereof capable of binding to the C-terminal region of human mature prolactin. The present disclosure also provides a method for measuring prolactin in a biological sample using the antibody pair of the present disclosure.

**EP 4 755 911 A2**

**Description**

**CROSS REFERENCE**

This application claims benefit of priority of Chinese Patent Application No. 202411798937.8 filed on December 06, 2024, the content of which is incorporated herein by reference in its entirety.

**TECHNICAL FIELD**

The present disclosure relates to an antibody pair for binding to human mature prolactin and uses thereof.

**BACKGROUND ART**

Prolactin (PRL), also known as "lactogen" or "pituitary prolactin", is a polypeptide hormone secreted by eosinophils of the anterior pituitary gland. Mature (signal peptide-removed) prolactin consists of 199 amino acids (SEQ ID NO: 1) and has a molecular weight of about 23 KD. Since PRL is the only biochemical indicator of hyperprolactinemia, it can be used clinically for the diagnosis of hyperprolactinemia. Increased PRL may also lead to symptoms such as oligomenorrhea or even amenorrhea, infertility, habitual abortion, osteopenia, galactorrhea in women, and impotence and infertility in men. When tumors occur, it can cause headaches, visual field defects, nausea, vomiting, abnormal appetite, drowsiness, coma, etc.

PRL exists in the human body in the following three forms:

- monomeric PRL: which accounts for about 80% to 90% of the total PRL, and has the highest biological and immune activity;

- dimeric PRL: which has a molecular weight of about 50KD, accounts for about 8% to 20% of the total PRL, and has relatively low biological and immune activity;

- macroprolactin: which is a complex of autoantibody IgG and PRL, has a molecular weight of greater than 100KD, accounts for about 1% to 5% of the total PRL, and has low or no biological and immune activity.

A discrepancy between clinical symptoms and serum prolactin (PRL) levels is often observed in practice due to the polymorphism of PRL in the systemic circulation, its varying proportions, and differences in biological activity. Specifically, macroprolactin has a high molecular weight, which prevents it from freely crossing the capillary wall and binding to target receptors. Consequently, it exhibits low or no biological activity in vivo. However, due to its long half-life, macroprolactin tends to accumulate in the bloodstream, creating a false impression of elevated PRL levels. The problem is that currently available commercial PRL assay kits cannot specifically measure monomeric PRL. This leads to the erroneous detection of largely biologically inactive macroprolactin, accumulated in the circulation, as bioactive monomeric PRL. As a result, healthy individuals may be misdiagnosed with hyperprolactinemia and potentially even subjected to inappropriate treatment.

Methods have also been developed in this field to eliminate the above-mentioned interference caused by macroprolactin, including gel filtration chromatography and polyethylene glycol (PEG) precipitation.

- The principle of gel filtration chromatography is to separate only monomeric PRL from total PRL by gel filtration chromatography, thereby determining the true level of monomeric PRL in human blood circulation. This method has reliable results and is considered to be the "gold standard" that can completely eliminate the above-mentioned interference caused by macroprolactin. However, it also has obvious disadvantages such as cumbersome and time-consuming operation, the need for specific equipment resources, high technical skill requirements, and difficulty in laboratory implementation.

- The principle of PEG precipitation method is to precipitate macroprolactin with larger molecular weight from total PRL by PEG precipitation, and the precipitated macroprolactin is removed by centrifugation to obtain the supernatant. This method is faster than gel filtration chromatography, but it is also cumbersome and slightly less reliable.

The present disclosure aims to develop a method for the specific determination of monomeric PRL that is faster than gel filtration chromatography and PEG precipitation.

## SUMMERY

The present disclosure includes the following contents:

Embodiment 1. An antibody pair capable of binding to human mature prolactin, wherein the antibody pair comprises:

a first monoclonal antibody or antigen-binding fragment thereof, which is capable of binding to the N-terminal region of human mature prolactin, and

a second monoclonal antibody or antigen-binding fragment thereof, which is capable of binding to the C-terminal region of human mature prolactin, and

the human mature prolactin consists of the amino acid sequence as set forth in SEQ ID NO: 1.

Embodiment 2. The antibody pair according to Embodiment 1, wherein the first monoclonal antibody or antigen-binding fragment thereof is capable of binding to a region within amino acids 1 to 13 of SEQ ID NO: 1, and/or the second monoclonal antibody or antigen-binding fragment thereof is capable of binding to a region within amino acids 183 to 199 of SEQ ID NO: 1.

Embodiment 3. The antibody pair according to Embodiment 2, wherein the region within amino acids 1 to 13 of SEQ ID NO: 1 comprises at least amino acids 1 to 3 and 10 to 13, and optionally comprises one or more conservative amino acid insertions, deletions or substitutions in the portion other than amino acids 1 to 3 and 10 to 13, and/or the region within amino acids 183 to 199 of SEQ ID NO: 1 comprises at least amino acids 184 to 186 and 199, and optionally comprises one or more conservative amino acid insertions, deletions or substitutions in the portion other than amino acids 184 to 186 and 199.

Embodiment 4. The antibody pair according to Embodiment 1, wherein

the first monoclonal antibody or antigen-binding fragment thereof comprises:

a heavy chain variable region, which comprises:

CDR1 comprising the amino acid sequence of GFIFX$_1$WYV,

CDR2 comprising the amino acid sequence of ISGGX$_2$SHT, and

CDR3 comprising the amino acid sequence of TRHSPYDHGDFGY, and

a light chain variable region, which comprises:

CDR1 comprising the amino acid sequence of QTLVHSLGNTY,

CDR2 comprising the amino acid sequence of KVSIA, and

CDR3 comprising the amino acid sequence of SQTTHVPTF; and/or the second monoclonal antibody or antigen-binding fragment thereof comprises:

a heavy chain variable region, which comprises:

CDR1 comprising the amino acid sequence of GASITSDYA,

CDR2 comprising the amino acid sequence of ISFGGGHT, and

CDR3 comprising the amino acid sequence of ARHSPNDHGDTNF, and

a light chain variable region, which comprises:

CDR1 comprising the amino acid sequence of QDISX$_3$NG,

CDR2 comprising the amino acid sequence of KTSX$_4$V, and

CDR3 comprising the amino acid sequence of QQGNTVPLT,

wherein,

X$_1$ is selected from the group consisting of: S, T, A or G;

X$_2$ is selected from the group consisting of: D, E, Q or N;

X$_3$ is selected from the group consisting of: D or E; and/or

X$_4$ is selected from the group consisting of: G, A, S or T;

preferably wherein:

X$_1$ is S or A;

X$_2$ is D or Q;

X$_3$ is D or E; and/or

X$_4$ is G, A or S.

Embodiment 5. The antibody pair according to Embodiment 4, wherein

the first monoclonal antibody or antigen-binding fragment thereof comprises:

a heavy chain variable region, which comprises an amino acid sequence selected from SEQ ID NOs: 5 and 32 to 46, and

a light chain variable region, which comprises an amino acid sequence as set forth in SEQ ID NO: 10; and/or

the second monoclonal antibody or antigen-binding fragment thereof comprises:

a heavy chain variable region, which comprises an amino acid sequence as set forth in SEQ ID NO: 15, and

a light chain variable region, which comprises an amino acid sequence selected from SEQ ID NOs: 20 and 53 to 59.

Embodiment 6. A monoclonal antibody or antigen-binding fragment thereof capable of binding to the N-terminal region of human mature prolactin, which comprises:

a heavy chain variable region, which comprises:

CDR1, which comprises the amino acid sequence of GFIFX$_1$WYV,

CDR2, which comprises the amino acid sequence of ISGGX$_2$SHT, and

CDR3, which comprises the amino acid sequence of TRHSPYDHGDFGY, and

a light chain variable region, which comprises:

CDR1, which comprises the amino acid sequence of QTLVHSLGNTY,

CDR2, which comprises the amino acid sequence of KVSIA, and

CDR3, which comprises the amino acid sequence of SQTTHVPTF,

wherein,

X$_1$ is selected from the group consisting of: S, T, A or G;

X$_2$ is selected from the group consisting of: D, E, Q or N;

preferably wherein:

X$_1$ is S or A; and

X$_2$ is D or Q.

Embodiment 7. The monoclonal antibody or antigen-binding fragment thereof according to Embodiment 6, which comprises:

a heavy chain variable region, which comprises an amino acid sequence selected from SEQ ID NOs: 5 and 32 to 46, and

a light chain variable region, which comprises an amino acid sequence as set forth in SEQ ID NO: 10.

Embodiment 8. A monoclonal antibody or antigen-binding fragment thereof capable of binding to the C-terminal region of human mature prolactin, which comprises:

a heavy chain variable region, which comprises:

CDR1, which comprises the amino acid sequence of GASITSDYA,
CDR2, which comprises the amino acid sequence of ISFGGGHT, and
CDR3, which comprises the amino acid sequence of ARHSPNDHGDTNF, and

a light chain variable region, which comprises:

CDR1, which comprises the amino acid sequence of QDISX$_3$NG,

CDR2, which comprises the amino acid sequence of KTSX$_4$V, and

CDR3, which comprises the amino acid sequence of QQGNTVPLT,

wherein,

X$_3$ is selected from the group consisting of: D or E; and

X$_4$ is selected from the group consisting of: G, A, S or T;

preferably wherein:

X$_3$ is D or E; and

X$_4$ is G, A or S.

Embodiment 9. The monoclonal antibody or antigen-binding fragment thereof according to Embodiment 8, which comprises:

a heavy chain variable region, which comprises an amino acid sequence as set forth in SEQ ID NO: 15, and

a light chain variable region, which comprises an amino acid sequence selected from SEQ ID NOs: 20 and 53 to 59.

Embodiment 10. A method for determining prolactin in a biological sample, comprising:

(a) contacting a biological sample suspected of containing prolactin with the antibody pair according to any one of Embodiments 1 to 5, and

(b) quantifying the prolactin in the biological sample according to a signal generated in step (a).

Embodiment 11. The method according to Embodiment 10, wherein the prolactin in the biological sample is quantified according to a chemiluminescent signal generated in step (a).

Embodiment 12. The method according to Embodiment 10 or 11, wherein the determination is independent of isolating monomeric mature prolactin from the biological sample.

Embodiment 13. The method according to Embodiment 10 or 11, wherein

(1) before step (a), the biological sample is pre-divided into a first part and a second part:

(1A) the first part is not subjected to any treatment and serves as a first test sample; and

(1B) the second part is mixed with an equal volume of a solution containing polyethylene glycol, followed by vortexing and centrifugation to obtain a supernatant, which serves as a second test sample;

(2) determining the prolactin concentrations in the first test sample and the second test sample through steps (a) and (b) as a first concentration and a second concentration, respectively; and

(3) calculating the recovery rate using the following formula:

Recovery rate of prolactin (%) = (the second concentration $\times$ 2)/(the first concentration) $\times$ 100;

wherein the recovery rate is greater than 60%, preferably greater than 80%, and more preferably greater than 90%.

Embodiment 14. A method for producing a monoclonal antibody, which comprises immunizing an animal with the following peptide:

the N-terminal region of human mature prolactin, and/or

the C-terminal region of human mature prolactin,

the human mature prolactin consists of the amino acid sequence as set forth in SEQ ID NO: 1.

Embodiment 15. The method according to Embodiment 14, wherein

the N-terminal region of human mature prolactin is a region within amino acids 1 to 13 of SEQ ID NO: 1, and/or the C-terminal region of human mature prolactin is a region within amino acids 183 to 199 of SEQ ID NO: 1;

preferably wherein: the region within amino acids 1 to 13 of SEQ ID NO: 1 comprises at least amino acids 1 to 3 and 10 to 13, and optionally comprises one or more conservative amino acid insertions, deletions or substitutions in the part other than amino acids 1 to 3 and 10 to 13, and/or the region within amino acids 183 to 199 of SEQ ID NO: 1 comprises at least amino acids 184 to 186 and 199, and optionally comprises one or more conservative amino acid insertions, deletions or substitutions in the part other than amino acids 184 to 186 and 199.

Technical Effect

When measuring the concentration of monomeric PRL in a sample using an antibody pair comprising one antibody that binds to the N-terminal region (specifically, a region within aa1 to aa13 of SEQ ID NO: 1, with residues aa1-aa3 and aa10-aa13 being critical residues) of human mature PRL, and another antibody that binds to the C-terminal region (specifically, a region within aa183 to aa199 of SEQ ID NO: 1, with residues aa184-aa186 and aa199 being critical residues), the assay is less susceptible to interference from macroprolactin present in the sample. That is, it exhibits a low tendency to misidentify macroprolactin as monomeric PRL, thereby offering significant advantages for the specific determination of monomeric PRL in samples.

## BRIEF DESCRIPTION OF THE DRAWINGS

FIG. 1A shows the schematic diagram of the three-dimensional structure of prolactin.

FIG. 1B shows the design of multiple continuous peptide fragments of about 30 amino acids (30 to 35 amino acids) covering the entire sequence of mature human PRL.

FIG. 2A shows the comparison of the recovery rate of PRL after PEG precipitation between products of the present disclosure and commercially available products.

FIG. 2B shows the comparison of products of the present disclosure and commercial products based on a dot-line graph of the concentration of monomeric PRL in samples prepared by gel filtration chromatography.

FIG. 2C shows the comparison of products of the present disclosure and control products.

FIG. 3 shows the PRL measurement effects of variant products 1 to 4.

## DETAILED DESCRIPTION

Definition of Terms

The terms used in this specification shall be construed as having meanings commonly used in the art, unless otherwise specified.

As used herein, the term "antibody" refers to an immunoglobulin molecule that can specifically bind to a target via at least one antigen recognition site located in the variable region of the immunoglobulin molecule. The "antibody" used herein includes not only complete (i.e., full-length) antibodies, but also antigen-binding fragments thereof (e.g., Fab, Fab', $F(ab')_2$, Fv, scFv, Fd, CDR fragments), variants thereof, fusion proteins comprising antibodies, humanized antibodies, chimeric antibodies, double antibodies, linear antibodies, single-chain antibodies, multispecific antibodies (e.g., bispecific antibodies), and any other modified configurations of immunoglobulin molecules containing antigen recognition sites of desired specificity, including antibody glycosylation variants, antibody amino acid sequence variants, and covalently modified antibodies. Antibodies can be derived from any mammals, including but not limited to humans, monkeys, pigs, horses, rabbits, dogs, cats, rats, mice, etc., or other animals such as birds (e.g., chickens), fish (e.g., sharks), and camels (e.g., llamas).

Typically, a complete or full-length antibody contains two heavy chains and two light chains. Each heavy chain contains a heavy chain variable region (VH) and a heavy chain constant region (CH); each light chain contains a light chain variable region (VL) and a light chain constant region (CL). Full-length antibodies can be any class of antibodies, such as IgD, IgE, IgG, IgA, or IgM (or subclasses of the above), but antibodies do not need to belong to any particular class. Immunoglobulins can be assigned to different classes based on their antibody heavy chain constant region amino acid sequences. Typically, there are five major classes of immunoglobulins: IgA, IgD, IgE, IgG, and IgM, and several of these classes can be further divided into subclasses, such as IgGl, IgG2, IgG3, IgG4, IgAl, and IgA2.

As used herein, the term "antigen-binding fragment" or "antigen-binding portion" refers to a portion or region of a complete antibody molecule that is responsible for binding to an antigen. The antigen-binding portion may comprise a heavy chain variable region (VH), a light chain variable region (VL), or both. Each of VH and VL typically comprises three complementary determining regions CDR1, CDR2, and CDR3. Non-limiting examples of antigen-binding fragment include Fab, Fab', $F(ab')_2$, Fd, Fv, complementary determining region (CDR) fragments, scFv, diabody, single domain antibody, chimeric antibody, linear antibody, nanobody (technology from Domantis), probody, and such polypeptides, which comprises at least a portion of antibody that is sufficient to confer the specific antigen binding ability to the polypeptide.

As used herein, the term "vector" refers to a nucleic acid vehicle into which a polynucleotide can be inserted. When a vector enables the expression of a protein encoded by the inserted polynucleotide, the vector is referred to as an expression vector. A vector can be introduced into a host cell by transformation, transduction or transfection, so that the genetic material elements carried by the vector can be expressed in the host cell. Vectors are well known to those skilled in the art and include, but are not limited to: plasmids; phagemids; cosmids; artificial chromosomes, such as yeast artificial chromosomes (YAC), bacterial artificial chromosomes (BAC) or P1-derived artificial chromosomes (PAC); phages such as λ phage or M13 phage and animal viruses. Animal viruses that can be used as vectors include, but are not limited to, retroviruses (including lentiviruses), adenoviruses, adeno-associated viruses, herpes viruses (e.g., herpes simplex virus), poxviruses, baculoviruses, papillomaviruses, papovavirus (e.g., SV40). A vector may comprise a variety of elements that control expression, including, but not limited to, promoter sequence, transcription initiation sequence, enhancer sequence, selection element, and reporter gene. In addition, the vector may comprise a replication initiation site.

As used herein, the term "host cell" refers to a cell containing a target protein of interest (e.g., the antibody or antigen-binding fragment thereof provided in any embodiment of the present disclosure). The target protein (e.g., the antibody or antigen-binding fragment thereof provided in any embodiment of the present disclosure) can be encoded by a polynucleotide, preferably an isolated polynucleotide. The polynucleotide can be present in the host cell in the following forms: (i) freely dispersed by itself, (ii) integrated into a recombinant vector, or (iii) integrated into a host cell genome or mitochondrial DNA. The host cell can be used to amplify and express a polynucleotide encoding a target protein of interest (e.g., the antibody or antigen-binding fragment thereof provided in any embodiment of the present disclosure). In a specific embodiment, the host cell can be selected from the group consisting of microbial host cells such as bacterial or yeast host cells, plant host cells, insect host cells or mammalian host cells, etc.

As used herein, the term "detection label" used in the present disclosure refers to a label that can be detected directly or in certain conditions (e.g., providing illumination conditions, adding a color developing reagent, an enzyme, etc.). The detection label is directly or indirectly coupled to the antibody or antigen-binding fragment thereof of the present disclosure through a covalent bond or a non-covalent bond. The detection label theoretically has no effect or little effect on the protein structure, function, and activity of the antibody or antigen-binding fragment thereof. In some embodiments, the detection label has no effect on the function of the antibody or antigen-binding fragment thereof coupled thereto. Such labels are readily known to those skilled in the art, for example, the detection label may be selected from the group consisting of radioactive labels (e.g., radioisotopes), fluorescent labels (e.g., fluorescein, phenanthridine dyes), chemiluminescent labels (e.g., acridinium ester compounds, cyanine dyes, luminol, isoluminol), biotin, colloidal gold, electrochemiluminescent labels (e.g., terpyridine ruthenium), quantum dots (e.g., gold quantum dots, CdSe quantum dots, ZnCdSe quantum dots, etc.) or enzymes (e.g., HRP (horse radish peroxidase), alkaline phosphatase, β-galactosidase).

As used herein, the term "double antibody sandwich method" specifically refers to the enzyme-linked immunosorbent assay (ELISA) double antibody sandwich assay, which is an immunoassay technique commonly used in which an antigen is bound to two different antibodies. The antibodies separately bind to two different epitopes in the antigen that do not overlap or interfere. In this assay, a sandwich comprising a capture antibody, an antigen, and a detection antibody is formed, whereby the antigen bridges the two antibodies bound thereto.

As used herein, the term "conservative amino acid modifications" is intended to refer to amino acid modifications that do not significantly affect or alter the expected properties or characteristics of a protein/polypeptide containing the amino acid sequence. Such conservative modifications include amino acid insertions, deletions or substitutions. Modifications can be introduced into the protein/polypeptide of the disclosure by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine).

The monoclonal antibody or antigen-binding fragment thereof of the present disclosure is a monoclonal antibody or antigen-binding fragment thereof having CDR or heavy chain variable region/light chain variable region as described in the description. The antibody or antibody fragment may also be derived from any class (e.g., IgG, IgE, IgM, IgD or IgA) or subclass of immunoglobulin molecule, for example, obtained from any species including mouse, rat, rabbit, pig, hamster, camel, alpaca, goat or human. In the present disclosure, the antigen-binding fragment may be selected from the group consisting of: Fab, Fab', F(ab')$_2$, scFv and dsFv, etc., and their definitions follow the common definitions in the art. In one embodiment, the monoclonal antibody of the present disclosure comprises a constant region derived from mammalian (e.g., mouse, rat, rabbit, sheep, monkey, or human) immunoglobulin. In a further embodiment, the monoclonal antibody of the present disclosure comprises a heavy chain constant region of IgG, e.g., IgG1, IgG2, IgG3 or IgG4. In a further embodiment, the monoclonal antibody of the present disclosure comprises a κ or λ type light chain constant region.

The monoclonal antibodies in the antibody pair of the present disclosure may be produced by any monoclonal antibody production process known in the art. In a specific embodiment, the monoclonal antibodies in the antibody pair of the present disclosure may be recovered from cloned hybridoma cells using methods such as protein A/G affinity chromatography and ion exchange.

PRL and epitopes thereon to be bound by the monoclonal antibodies of the antibody pair of the present disclosure

The amino acid sequence of the prolactin precursor of *Homo sapiens* is included in the accession number ACT52574.1 of GenBank (https://www.ncbi.nlm.nih.gov/protein/ACT52574.1), which has a total length of 227 amino acids (227aa), of which aa1-28 is the amino acid sequence of the signal peptide, and aa29-227 is the amino acid sequence of mature human PRL (SEQ ID NO: 1).

[Amino acid sequence of human mature PRL (SEQ ID NO: 1)]

LPICPGGAAR CQVTLRDLFD RAVVLSHYIH NLSSEMFSEF DKRYTHGRGF ITKAINSCHT SSLATPEDKE QAQQMNQKDF LSLIVSILRS WNEPLYHLVT EVRGMQEAPE AILSKAVEIE EQTKRLLEGM ELIVSQVHPE TKENEIYPVW SGLPSLQMAD EESRLSAYYN LLHCLRRDSH KIDNYLKLLK CRIIHNNNC

In the present disclosure, it was first discovered that, by performing a sandwich immunoassay using an antibody pair consisting of antibody 1 and antibody 2, which are capable of binding to the N-terminal region (or a conservative variant thereof) and the C-terminal region (or a conservative variant thereof) of human mature PRL (SEQ ID NO: 1) respectively (hereinafter sometimes referred to as the "antibody pair of the present disclosure"), monomeric PRL can be bound with significantly higher specificity from total PRL in the blood circulation.

In one embodiment, the antibody 1 is capable of binding to a region within amino acids 1 to 13 of SEQ ID NO: 1 (SEQ ID NO: 2), and the antibody 2 is capable of binding to a region within amino acids 183 to 199 of SEQ ID NO: 1 (SEQ ID NO: 3).

[Amino acids 1 to 13 of human mature PRL (SEQ ID NO: 2)]

LPICPGGAARCQV

In one embodiment, the region within amino acids 1 to 13 of SEQ ID NO: 1 comprises at least amino acids 1 to 3 and 10 to 13. In one embodiment, in the region within amino acids 1 to 13 of SEQ ID NO: 1 (SEQ ID NO: 2), the portion other than amino acids 1 to 3 and 10 to 13 of SEQ ID NO: 1 (the underlined amino acid residues) may have one or more conservative amino acid insertions, deletions or substitutions.

[Amino acids 183 to 199 of human mature PRL (SEQ ID NO: 3)]

DNYLKLLKCR IIHNNNC

In one embodiment, the region within amino acids 183 to 199 of SEQ ID NO: 1 comprises at least amino acids 184 to 186 and 199. In one embodiment, in the region within amino acids 183 to 199 of SEQ ID NO: 1 (SEQ ID NO: 3), the portion other than amino acids 184 to 186 and 199 (the underlined amino acid residues) of SEQ ID NO: 1 may have one or more conservative amino acid insertions, deletions or substitutions.

In one embodiment, the amino acid sequences of the CDRs of the heavy chain variable regions and the light chain variable regions of the above-mentioned antibody 1 and antibody 2 are as follows:

| | | Antibody 1 | | Antibody 2 | |
|---|---|---|---|---|---|
| Heavy chain | CDR1 | GFIFX$_1$WYV | SEQ ID NO: 24 | GASITSDYA | SEQ ID NO: 16 |
| | CDR2 | ISGGX$_2$SHT | SEQ ID NO: 28 | ISFGGGHT | SEQ ID NO: 17 |
| | CDR3 | TRHSPYDHGDFGY | SEQ ID NO: 8 | ARHSPNDHGDTNF | SEQ ID NO: 18 |
| Light chain | CDR1 | QTLVHSLGNTY | SEQ ID NO: 11 | QDISX$_3$NG | SEQ ID NO: 47 |
| | CDR2 | KVSIA | SEQ ID NO: 12 | KTSX$_4$V | SEQ ID NO: 49 |
| | CDR3 | SQTTHVPTF | SEQ ID NO: 13 | QQGNTVPLT | SEQ ID NO: 23 |

wherein,

• X$_1$ is selected from the group consisting of S, T, A or G, preferably S or A;

| $X_1$ | Heavy chain CDR1 in antibody 1 | SEQ ID NO: 24 |
|---|---|---|
| S | GFIFSWYV | SEQ ID NO: 6 |
| T | GFIFTWYV | SEQ ID NO: 25 |
| A | GFIFAWYV | SEQ ID NO: 26 |
| G | GFIFGWYV | SEQ ID NO: 27 |

• $X_2$ is selected from the group consisting of D, E, Q or N, preferably D or Q;

| $X_2$ | Heavy chain CDR2 in antibody 1 | SEQ ID NO: 28 |
|---|---|---|
| D | ISGGDSHT | SEQ ID NO: 7 |
| E | ISGGESHT | SEQ ID NO: 29 |
| Q | ISGGQSHT | SEQ ID NO: 30 |
| N | ISGGNSHT | SEQ ID NO: 31 |

• $X_3$ is selected from the group consisting of D or E; and

| $X_3$ | Light chain CDR1 in antibody 2 | SEQ ID NO: 47 |
|---|---|---|
| D | QDISDNG | SEQ ID NO: 21 |
| E | QDISENG | SEQ ID NO: 48 |

• $X_4$ is selected from: G, A, S or T.

| $X_4$ | Light chain CDR2 in antibody 2 | SEQ ID NO: 49 |
|---|---|---|
| G | KTSGV | SEQ ID NO: 22 |
| A | KTSAV | SEQ ID NO: 50 |
| S | KTSSV | SEQ ID NO: 51 |
| T | KTSTV | SEQ ID NO: 52 |

Therefore, the specific sequences of the variable regions of antibody 1 can be as follows:

| Antibody 1 | $X_1$ | $X_2$ | Heavy chain variable region | Light chain variable region |
|---|---|---|---|---|
| Antibody 1a | S | D | SEQ ID NO: 5 | SEQ ID NO: 10 |
| Antibody 1b | S | E | SEQ ID NO: 32 | SEQ ID NO: 10 |
| Antibody 1c | S | Q | SEQ ID NO: 33 | SEQ ID NO: 10 |
| Antibody 1d | S | N | SEQ ID NO: 34 | SEQ ID NO: 10 |
| Antibody 1e | T | D | SEQ ID NO: 35 | SEQ ID NO: 10 |
| Antibody 1f | T | E | SEQ ID NO: 36 | SEQ ID NO: 10 |
| Antibody 1g | T | Q | SEQ ID NO: 37 | SEQ ID NO: 10 |
| Antibody 1h | T | N | SEQ ID NO: 38 | SEQ ID NO: 10 |
| Antibody 1i | A | D | SEQ ID NO: 39 | SEQ ID NO: 10 |
| Antibody 1j | A | E | SEQ ID NO: 40 | SEQ ID NO: 10 |
| Antibody 1k | A | Q | SEQ ID NO: 41 | SEQ ID NO: 10 |

(continued)

| Antibody 1 | $X_1$ | $X_2$ | Heavy chain variable region | Light chain variable region |
|---|---|---|---|---|
| Antibody 1l | A | N | SEQ ID NO: 42 | SEQ ID NO: 10 |
| Antibody 1m | G | D | SEQ ID NO: 43 | SEQ ID NO: 10 |
| Antibody 1n | G | E | SEQ ID NO: 44 | SEQ ID NO: 10 |
| Antibody 1o | G | Q | SEQ ID NO: 45 | SEQ ID NO: 10 |
| Antibody 1p | G | N | SEQ ID NO: 46 | SEQ ID NO: 10 |

* As long as they contain the same HCDR1-3 and LCDR1-3 sequences, the sequences of the heavy chain variable regions and the light chain variable regions are not limited to the above sequences.

The specific sequences of the variable regions of antibody 2 may be as follows:

| Antibody 2 | $X_3$ | $X_4$ | Heavy chain variable region | Light chain variable region |
|---|---|---|---|---|
| Antibody 2a | D | G | SEQ ID NO: 15 | SEQ ID NO: 20 |
| Antibody 2b | D | A | SEQ ID NO: 15 | SEQ ID NO: 53 |
| Antibody 2c | D | S | SEQ ID NO: 15 | SEQ ID NO: 54 |
| Antibody 2d | D | T | SEQ ID NO: 15 | SEQ ID NO: 55 |
| Antibody 2e | E | G | SEQ ID NO: 15 | SEQ ID NO: 56 |
| Antibody 2f | E | A | SEQ ID NO: 15 | SEQ ID NO: 57 |
| Antibody 2g | E | S | SEQ ID NO: 15 | SEQ ID NO: 58 |
| Antibody 2h | E | T | SEQ ID NO: 15 | SEQ ID NO: 59 |

* As long as they contain the same HCDR1-3 and LCDR1-3 sequences, the sequences of the heavy chain variable regions and the light chain variable regions are not limited to the above sequences.

In one embodiment, the antibody 1 is a capture antibody and the antibody 2 is a detection antibody; in another embodiment, the antibody 2 is a capture antibody and the antibody 1 is a detection antibody. In a preferred embodiment, the antibody 1 is a capture antibody and the antibody 2 is a detection antibody.

In one embodiment, the antibody 1 is Antibody 1a, 1c, 1i or 1k. In one embodiment, the antibody 2 is Antibody 2a, 2c or 2f. In a further embodiment, the antibody pair of the present disclosure comprises:

Antibody 1a as the capture antibody, and Antibody 2a as the detection antibody;

Antibody 2a as the capture antibody, and Antibody 1a as the detection antibody;

Antibody 1c as the capture antibody, and Antibody 2a as the detection antibody;

Antibody 1i as the capture antibody, and Antibody 2c as the detection antibody;

Antibody 1k as the capture antibody, and Antibody 2f as the detection antibody; or

Antibody 1a as the capture antibody, and Antibody 2c as the detection antibody;

wherein:

Antibody 1a comprises: HCDR1, HCDR2, HCDR3 as shown in SEQ ID NOs: 6, 7, 8; LCDR1, LCDR2, LCDR3 as shown in SEQ ID NOs: 11, 12, 13;

Antibody 1c comprises: HCDR1, HCDR2, HCDR3 as shown in SEQ ID NOs: 6, 30, 8, LCDR1, LCDR2, LCDR3 as shown in SEQ ID NOs: 11, 12, 13;

Antibody 1i comprises: HCDR1, HCDR2, HCDR3 as shown in SEQ ID NOs: 26, 7, 8, LCDR1, LCDR2, LCDR3 as shown in SEQ ID NOs: 11, 12, 13;

Antibody 1k comprises: HCDR1, HCDR2, HCDR3 as shown in SEQ ID NOs: 26, 30, 8, LCDR1, LCDR2, LCDR3 as shown in SEQ ID NOs: 11, 12, 13;

Antibody 2a comprises: HCDR1, HCDR2, HCDR3 as shown in SEQ ID NOs: 16, 17, 18; LCDR1, LCDR2, LCDR3 as shown in SEQ ID NOs: 21, 22, 23;

Antibody 2c comprises: HCDR1, HCDR2, HCDR3 as shown in SEQ ID NOs: 16, 17, 18; LCDR1, LCDR2, LCDR3 as shown in SEQ ID NOs: 21, 51, 23;

Antibody 2f comprises: HCDR1, HCDR2, HCDR3 as shown in SEQ ID NOs: 16, 17, 18; LCDR1, LCDR2, LCDR3 as shown in SEQ ID NOs: 48, 50, 23.

In one embodiment, the antibody 1 of the present disclosure can be obtained by immunizing an animal with the following polypeptides:

• a peptide comprising the amino acid sequence as set forth in SEQ ID NO: 2, or

• a peptide comprising an amino acid sequence comprising one or more conservative amino acid insertions, deletions or substitutions in the portion other than aa1-3 and aa10-13 as compared with the amino acid sequence as set forth in SEQ ID NO: 2.

In one embodiment, the antibody 1 of the present disclosure is obtained by immunizing an animal with the following polypeptides:

• a peptide consisting of the amino acid sequence as set forth in SEQ ID NO: 2, or

• a peptide consisting of an amino acid sequence comprising one or more conservative amino acid insertions, deletions or substitutions in the portion other than aa1-3 and aa10-13 as compared with the amino acid sequence as set forth in SEQ ID NO: 2.

In one embodiment, the antibody 2 of the present disclosure is obtained by immunizing an animal with the following polypeptides:

• a peptide comprising the amino acid sequence as set forth in SEQ ID NO: 3, or

• a peptide comprising an amino acid sequence comprising one or more conservative amino acid insertions, deletions or substitutions in the portion other than aa2-4 and aa17 (numbered according to SEQ ID NO: 3) as compared with the amino acid sequence as set forth in SEQ ID NO: 3.

In one embodiment, the antibody 2 of the present disclosure is obtained by immunizing an animal with the following polypeptides:

• a peptide consisting of the amino acid sequence as set forth in SEQ ID NO: 3, or

• a peptide consisting of an amino acid sequence comprising one or more conservative amino acid insertions, deletions or substitutions in the portion other than aa2-4 and aa17 (numbered according to SEQ ID NO: 3) as compared with the amino acid sequence as set forth in SEQ ID NO: 3.

In one embodiment, the present disclosure relates to a method for producing a monoclonal antibody, which comprises immunizing an animal with the N-terminal region of human mature prolactin and/or the C-terminal region of human mature prolactin, wherein the human mature prolactin consists of the amino acid sequence as set forth in SEQ ID NO: 1. In a further embodiment, the N-terminal region of human mature prolactin is a region within amino acids 1 to 13 of SEQ ID NO: 1, and/or the C-terminal region of human mature prolactin is a region within amino acids 183 to 199 of SEQ ID NO: 1. In one embodiment, the region within amino acids 1 to 13 of SEQ ID NO: 1 comprises at least amino acids 1 to 3 and 10 to 13, and/or, the region within amino acids 183 to 199 of SEQ ID NO: 1 comprises at least amino acids 184 to 186 and 199. In a further embodiment, in the region within amino acids 1 to 13 of SEQ ID NO: 1, there may be one or more conservative amino acid insertions, deletions or substitutions in the portion other than positions 1 to 3 and positions 10 to 13 of SEQ ID NO: 1, and/or in the region within amino acids 183 to 199 of SEQ ID NO: 1, there may be one or more conservative amino acid insertions, deletions or substitutions in the portion other than positions 184 to 186 and position 199 of SEQ ID NO: 1.

In the above embodiment, the one or more conservative amino acid insertions, deletions or substitutions include but are not limited to at least one, at least two, at least three, at least four, or at least five conservative amino acid insertions, deletions or substitutions.

In one embodiment, the present disclosure also relates to a polynucleotide encoding the heavy chain, heavy chain variable region, light chain and light chain variable region of each antibody in the above-mentioned antibody pair. In a further embodiment, the polynucleotide encodes the monoclonal antibody or antigen-binding fragment thereof capable of binding to the N-terminal region of human mature prolactin of the present disclosure. In a further embodiment, the polynucleotide encodes the monoclonal antibody or antigen-binding fragment thereof capable of binding to the C-terminal region of human mature prolactin of the present disclosure. In one embodiment, the present disclosure also relates to an expression vector comprising the above-mentioned polynucleotide. In one embodiment, the present disclosure also relates to a host cell comprising the polynucleotide or the expression vector.

In the present disclosure, the heavy chain/light chain variable region or CDRs thereof may "comprise" the amino acid sequence as set forth in SEQ ID NO: ..., or consist of the amino acid sequence as set forth in SEQ ID NO: ... Similarly, the nucleotide sequence encoding the heavy chain/light chain variable region or CDRs thereof may "comprise" the nucleotide sequence as set forth in SEQ ID NO: ..., or consists of the nucleotide sequence as set forth in SEQ ID NO: ....

Determination of PRL

In one embodiment, the antibody pair of the present disclosure is used to determine prolactin in a biological sample. In a further embodiment, the determination of prolactin in a biological sample can be performed by various methods known in the art. Suitable assays include, but are not limited to, enzyme-linked immunoassay, radioimmunoassay, fluorescent immunoassay, chemiluminescence immunoassay, etc. In a preferred embodiment, the determination of prolactin in a biological sample can be performed by a double-antibody sandwich chemiluminescence immunoassay (chemiluminescence immunoassay, CLIA).

The basic principle of the double-antibody sandwich method is that: an antibody immobilized on a solid phase carrier is used as a capture antibody to capture an antigen to be detected, and then another antibody labeled with an enzyme is used as a detection antibody to bind to the antigen to be detected captured by the capture antibody. After adding a chromogenic or chemiluminescent substrate for the enzyme, the intensity of the resulting color or chemiluminescence is directly proportional to the concentration of the antigen to be detected.

In the present disclosure, the solid phase carrier used to immobilize the capture antibody is not particularly limited, and can be, but not limited to, magnetic beads, polyvinyl chloride, polystyrene, polyacrylamide and cellulose. In a further embodiment, the solid phase carrier is magnetic bead, e.g., superparamagnetic microbeads.

In the present disclosure, the detection antibody bears a detection label, e.g., radioactive label, fluorescent label, chemiluminescent label, biotin, colloidal gold, electrochemiluminescent label, quantum dot or enzyme. In one embodiment, the detection label is selected from enzyme. In the present disclosure, the enzyme used to label the detection antibody is not particularly limited, and may be, but not limited to, alkaline phosphatase, horseradish peroxidase, etc.

In the present disclosure, the choice of chromogenic or chemiluminescent substrate depends on the enzyme used to label the detection antibody. In one embodiment, when the detection antibody is labeled with alkaline phosphatase, any substrate that can develop color/generate chemiluminescence under the action of alkaline phosphatase may be used, and examples thereof include, but not limited to, 3-(2-spiroadamantane)-4-methoxy-4- (3-phosphoroxyacyl)-phenyl-1,2-dioxetane (AMPPD), etc. In one embodiment, AMPPD loses a phosphate group under the action of alkaline phosphatase to generate an unstable intermediate, the intermediate generates methyl m-oxybenzoate anion through intramolecular electron transfer, and chemiluminescence is generated when the excited methyl methyl m-oxybenzoate anion returns from the excited state to the ground state. In another embodiment, when the detection antibody is labeled with horseradish peroxidase, any substrate that can develop color under the action of horseradish peroxidase can be used, and examples thereof include, but not limited to, 3,3',5,5'-tetramethylbenzidine (TMB), etc.

In one embodiment, the present disclosure relates to a method for determining prolactin in a biological sample, which comprises:

(a) contacting a biological sample suspected of containing prolactin with the antibody pair of the present disclosure, and

(b) quantifying the prolactin in the biological sample based on the signal generated in step (a).

In a further embodiment, step (a) comprises: (i) contacting the biological sample with the capture antibody of the antibody pair to form an antibody-antigen complex; (ii) contacting the antibody-antigen complex with the detection antibody of the antibody pair to form an antibody-antigen-antibody complex; and (iii) detecting the antibody-antigen-antibody complex.

In a further embodiment, the antibody 1 is used as a capture antibody, and the antibody 2 is used as a detection antibody. In another embodiment, the antibody 2 is used as a capture antibody, and the antibody 2 is used as a detection antibody.

In a further embodiment, the prolactin is determined by a double-antibody sandwich chemiluminescent immunoassay. In a preferred embodiment, the prolactin in the biological sample is quantified according to the chemiluminescent signal.

In a further embodiment,

(1) before step (a), the biological sample is pre-divided into a first part and a second part:

(1A) the first part is not subjected to any treatment and serves as a first test sample; and

(1B) the second part is mixed with an equal volume of a solution containing polyethylene glycol, followed by vortexing and centrifugation to obtain a supernatant, which serves as a second test sample;

(2) determining the prolactin concentrations in the first test sample and the second test sample through steps (a) and (b) as a first concentration and a second concentration, respectively; and

(3) calculating the recovery rate using the following formula:

Recovery rate of prolactin (%) = (the second concentration $\times$ 2)/(the first concentration) $\times$ 100

wherein the recovery rate is greater than 60%, preferably greater than 80%, and more preferably greater than 90%.

In one embodiment, the biological sample to be determined for PRL can be selected from, but not limited to: whole blood, serum or plasma, etc.

In one embodiment, the present disclosure relates to use of the above-mentioned antibody pair and each antibody in the antibody pair in the manufacture of a kit for measuring prolactin in a biological sample.

In one embodiment, the present disclosure relates to a method for measuring prolactin in a biological sample comprising using antibody 1 and/or antibody 2 of the present disclosure to perform immunoassay on the sample. In a further embodiment, the immunoassay is double-antibody sandwich chemiluminescence immunoassay.

## EXAMPLES

Example 1: Acquisition of monoclonal antibodies and determination of epitopes on human mature PRL to which they bind

(1.1) Acquisition of monoclonal antibodies

The human mature PRL as set forth in SEQ ID NO: 1 was used as an immunogen and injected into Balb/c mice to stimulate their immune system to produce antibodies. B cells that produce antibodies were isolated from the spleen of the immunized mice. According to conventional methods, the isolated B cells were fused with SP2/0 myeloma cells to form hybridoma cells. Monoclonal clones that bound well to human mature PRL as an antigen were screened and cloned by limiting dilution method. Monoclonal antibodies were recovered from the hybridoma cells cloned as above by affinity chromatography using protein A.

The binding specificity of the obtained monoclonal antibodies to human mature PRL was detected, and antibodies with better binding specificity to human mature PRL were screened. Among which, two antibodies were named "antibody 1" and "antibody 2" (more specifically "antibody 1a" and "antibody 2a"), and the amino acid sequences of heavy chain variable regions CDR1-3 and light chain variable regions CDR1-3 are shown in the following table.

| | | Antibody 1 (more specifically "antibodyla") | | Antibody 2 (more specifically "antibody2a") | |
|---|---|---|---|---|---|
| | | Sequence | SEQ ID | Sequence | SEQ ID |
| Heavy chain | H | EVMSAESGGG LVKPGGSLKF SCAASWFGFS GFIFSWYVVW RQTPMERLFW VAISGGDSHT RFNISRNNAK NLTLYLMQSS LRSNATAMYY CWRTRHSPYD HGDFGYVGQG ATLTVSSAKT TAPSVYPLAP VCGDTTGSSV TLGCLVKGYF PEPVTLTWNS GSLSSGVHTF PAVLQSDLYT LSSSVTVTSS TWPSQSITCN VAHPASSTKV DKKIEPRGPT IKPCPPCKCP APNLLGGPSV FIFPPKIKDV LMISLSPIVT CVVVDVSEDD PDVQISWFVN NVEVHTAQTQ THREDYNSTL RVVSALPIQH QDWMSGKEFK CKVNNKDLPA PIERTISKPK GSVRAPQVYV LPPPEEEMTK KQVTLTCMVT DFMPEDIYVE WTNNGKTELN YKNTEPVLDS DGSYFMYSKL RVEKKNWVER NSYSCSVVHE GLHNHHTTKS FSRTPGK | 4 | EVMLVESGGG LVRPGGSLKL SCAASGFTFS GASITSDYAW VRQTPEKRLE WVAISFGGGH TRFTISRDNA KNTLYLQMSS LKSDDTAMYY CARARHSPND HGDTNFWGQG TTLAVSSAKT TPPSVYPLAP GSAAQTNSMV TLGCLVKGYF PEPVTVTWNS GSLSSGVHTF PAVLQSDLYT LSSSVTVPSS TWPSETVTCN VAHPASSTKV DKKIVPRDCG CKPCICTVPE VSSVFIFPPK PKDVLTITLT PKVTCVVVDI SKDDPEVQFS WFVDDVEVHT AQTQPREEQF NSTFRSVSEL PIMHQDWLNG KEFKCRVNSA AFPAPIEKTI SKTKGRPKAP QVYTIPPPKE QMAKDKVSLT CMITDFFPED ITVEWQWNGQ PAENYKNTQP IMDTDGSYFV YSKLNVQKSN WEAGNTFTCS VLHEGLHNHH TEKSLSHSPG K | 14 |
| | VH | EVMSAESGGG LVKPGGSLKF SCAASWFGFS GFIFSWYVVW RQTPMERLFW VAISGGDSHT RFNISRNNAK NLTLYLMQSS LRSNATAMYY CWRTRHSPYD HGDFGYVGQG ATLTVSS | 5 | EVMLVESGGG LVRPGGSLKL SCAASGFTFS GASITSDYAW VRQTPEKRLE WVAISFGGGH TRFTISRDNA KNTLYLQMSS LKSDDTAMYY CARARHSPND HGDTNFWGQG TTLAVSS | 15 |
| | CDR1 | GFIFSWYV | 6 | GASITSDYA | 16 |

EP 4 755 911 A2

(continued)

| | | Antibody 1 (more specifically "antibody1a") | | Antibody 2 (more specifically "antibody2a") | |
|---|---|---|---|---|---|
| | | Sequence | SEQ ID | Sequence | SEQ ID |
| | CDR2 | ISGGDSHT | 7 | ISFGGGHT | 17 |
| | CDR3 | TRHSPYDHGDFGY | 8 | ARHSPNDHGDTNF | 18 |
| Light chain | L | DAMVTQESAL TTSPGETVTL TCQTLVHSLG NTYWVQEKPD HLFTGLIGKV SIAGVPDRFS GSGSGTDFTL KISRVEAEDL GVYFCSQTTH VPTFFGGGTK LEIKRADAAP TVSIFPPSSE QLTSGGASVV CFLNNFYPKD INVKWKIDGS ERQNGVLNSW TDQDSKDSTY SMSSTLTLTK DEYERHNSYT CEATHKTSTS PIVKSFNRNE C | 9 | DVVMTQTPLS LPVSLGDQSS ISCQDISDNG WYLQKPVQSP KLLIYKTSGV GVPDCFSGSG SGTDFSLKIS RVETEDLGVY FCQQGNTVPL TFGGGTKLEI KRADAAPTVS IFPPSSEQLT SGGASVVCFL NNFYPKDINV KWKIDGSERQ NGVLNSWTDQ DSKDSTYSMS STLTLTKDEY ERHNSYTCEA THKTSTSPIV KSFNRNEC | 19 |
| | VL | DAMVTQESAL TTSPGETVTL TCQTLVHSLG NTYWVQEKPD HLFTGLIGKV SIAGVPDRFS GSGSGTDFTL KISRVEAEDL GVYFCSQTTH VPTFFGGGTK LEIK | 10 | DVVMTQTPLS LPVSLGDQSS ISCQDISDNG WYLQKPVQSP KLLIYKTSGV GVPDCFSGSG SGTDFSLKIS RVETEDLGVY FCQQGNTVPL TFGGGTKLEI K | 20 |
| | CDR1 | QTLVHSLGNTY | 11 | QDISDNG | 21 |
| | CDR2 | KVSIA | 12 | KTSGV | 22 |
| | CDR3 | SQTTHVPTF | 13 | QQGNTVPLT | 23 |

(1.2) Determination of epitopes on human mature PRL bound by monoclonal antibodies

For the antibodies with better binding specificity to human mature PRL screened in (1.1) (especially the above-mentioned antibody 1 and antibody 2), the epitopes on human mature PRL bound by these antibodies were analyzed as follows:

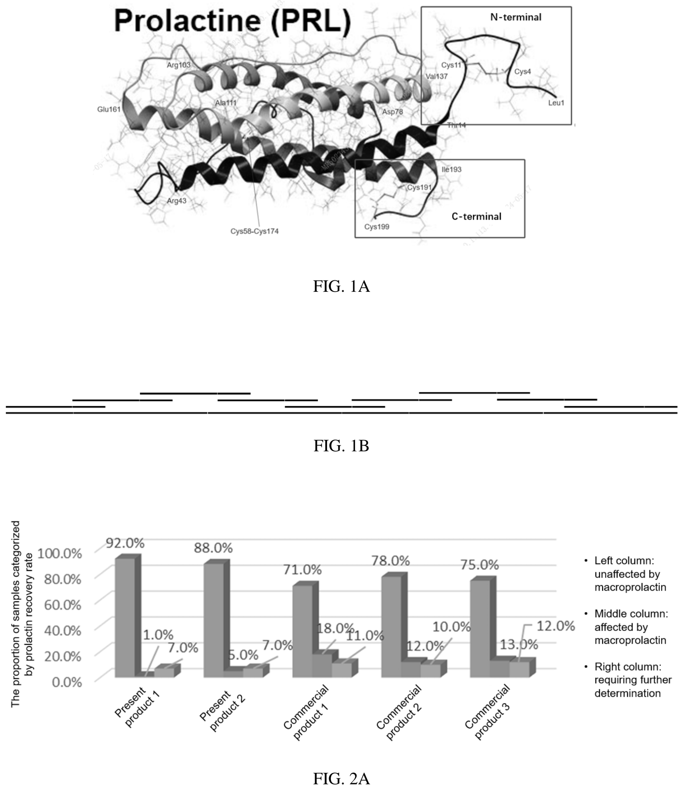

(a) As shown in FIG. 1b, based on the amino acid sequence of human mature PRL (SEQ ID NO: 1), multiple continuous peptide fragments of about 30aa (30-35aa) capable of covering its entire sequence were synthesized, in which the adjacent peptide fragments of about 30aa overlapped by about 10 amino acids between them. A competitive binding assay was performed between these peptide fragments of about 30aa and the full-length human mature PRL for binding to the aforementioned Antibody 1 and Antibody 2. The results demonstrated that:

- Antibody 1 preferentially bound to the peptide fragment corresponding to the N-terminal aa1-30 of human mature PRL (SEQ ID NO: 1);

- Antibody 2 preferentially bound to the peptide fragment corresponding to the C-terminal aa176-199 of human mature PRL (SEQ ID NO: 1).

(b) Further, based on the amino acid sequences of the N-terminal aal-30 peptide fragment and the C-terminal aa176-199 peptide fragment of human mature PRL, a series of shorter peptide fragments (e.g., 15aa, 13aa, 10aa, etc.) were synthesized to cover these regions, in which the adjacent peptide fragments overlapped by several amino acids. A competitive binding assay was performed between these shorter peptide fragments and the N-terminal aal-30 peptide fragment and the C-terminal aa176-199 peptide fragment of human mature PRL for binding to the aforementioned Antibody 1 and Antibody 2. The results demonstrated that:

- Antibody 1 preferentially bound to the N-terminal aa1-13 peptide fragment corresponding to human mature PRL (SEQ ID NO: 1), that was, Antibody 1 bound to the region of aa1-13 (SEQ ID NO: 2) of human mature PRL (SEQ ID NO: 1);

- Antibody 2 preferentially bound to the C-terminal aa183-199 peptide fragment corresponding to human mature PRL (SEQ ID NO: 1), that was, Antibody 2 bound to the region of aa183-199 (SEQ ID NO: 3) of mature human PRL (SEQ ID NO: 1).

(c) The deletion/substitution mutants of the N-terminal aa1-13 peptide fragment and the C-terminal aa183-199 peptide fragment of mature human PRL (SEQ ID NO: 1) were prepared by deletion/substitution of one or more amino acids. The binding properties of these deletion/substitution mutants to the above-mentioned Antibody 1 and Antibody 2 were measured. The results demonstrated that:

- The residues aa1-3 and aa10-13 of human mature PRL (SEQ ID NO: 1) (underlined amino acid residues in the following sequence) were the key residues in the epitope (aa1-13 of SEQ ID NO: 1 (SEQ ID NO: 2)) bound by Antibody 1:

    [aa1-13 of human mature PRL (SEQ ID NO: 2)]

    LPICPGGAARCQV

- The residues aa184-186 and aa199 of human mature PRL (SEQ ID NO: 1) (underlined amino acid residues in the following sequence) were the key residues in the epitope (aa183-199 of SEQ ID NO: 1 (SEQ ID NO: 3)) bound by Antibody 2:

    [aa183-199 of human mature PRL (SEQ ID NO: 3)]

    DNYLKLLKCRIIHNNNC

The above-mentioned antibody 1 and antibody 2 were used for further experiments.

Example 2: Determination of monomeric PRL in sample by antibody kit

(2.1) Antibody kit

The antibody kit as shown in the following table was prepared using antibody 1 and antibody 2 obtained in Example 1, which are referred to as "present product 1" and "present product 2".

| Antibody kit | Capture reagent | | Detection reagent | |
|---|---|---|---|---|
| | Capture antibody | Magnetic beads | Detection antibody | Label |
| Present Product 1 | Antibody 1 | Superparamagnetic microbeads | Antibody 2 | Alkaline phosphatase |
| Present Product 2 | Antibody 2 | | Antibody 1 | |

As a control antibody kit, the following anti-human mature PRL antibodies capable of binding to the non-N-terminal and non-C-terminal regions were used to form an antibody pair of capture antibody and detection antibody, which was referred to as "control product".

| Antibody kit | Capture reagent | | Detection reagent | |
|---|---|---|---|---|
| | Capture antibody | Magnetic beads | Detection antibody | Label |
| Control product 1 | Antibody α (binding to aa41-69 of mature human PRL) | Superparamagnetic microbeads | Antibody β (binding to aa121-151 of mature human PRL) | Alkaline phosphatase |
| Control product 2 | Antibody β (binding to aa121-151 of mature human PRL) | | Antibody α (binding to aa41-69 of mature human PRL) | |

As comparative antibody kits, commercial product 1 (Abbott), commercial product 2 (Roche) and commercial product 3 (Beckman) of the following compositions were used.

In the above antibody kits,

- The above capture antibody was coated on superparamagnetic microbeads to serve as a capture reagent.

- The above detection antibody was labeled with alkaline phosphatase to serve as a detection reagent.

(2.2) Samples

Initial samples: Human clinical serum samples were used as initial samples. These samples included: positive samples with PRL concentration values outside the normal range, normal samples with PRL concentration values within the normal range, and suspected samples with PRL concentration values suspected of being interfered by macroprolactin.

Samples prepared by gel filtration chromatography: The initial samples were centrifuged at 10,000 rpm for 10 min, the supernatant was collected, and the supernatant was passed through an Agilent advanceBio SEC 300A chromatographic column on a liquid chromatograph (Cat. 1260, Agilent) to collect only the monomeric PRL fraction as the sample prepared by gel filtration chromatography.

Samples prepared by PEG precipitation: The initial samples were mixed with a 25% PEG 6000 solution at a volume ratio of 1:1, and vortexed. The mixture was then centrifuged at 8,000 rpm for 5 min, and the supernatant was collected as the sample prepared by PEG precipitation.

(2.3) Determination of monomeric PRL in samples

(2.3.1) Determination of PRL in samples using "Present product 1" and "Present product 2" as well as "control product 1" and "control product 2"

The above samples were determined according to the standard procedure using present product 1 and present product 2 as well as control product 1 and control product 2 in a chemiluminescence analyzer (Mindray).

(2.3.2) Determination of PRL in samples using commercial products 1, 2 and 3

Commercial products 1, 2 and 3 were loaded onto their respective instruments and the above samples were determined according to their instructions.

(2.4) Evaluation criteria

(2.4.1) Determination of samples prepared by PEG precipitation

The recovery rate of PRL after PEG precipitation of each sample was calculated by the following formula:

Recovery rate of PRL after PEG precipitation (%) = (Concentration of monomeric PRL in sample prepared by PEG precipitation $\times$ 2)/(Concentration of PRL in initial sample) $\times$ 100

All the samples prepared by PEG precipitation (100%) were divided into the following groups:

- Samples with a recovery rate of >60% were defined as: samples whose PRL determination results were not interfered by macroprolactin (left columns, accounting for x%);

- Samples with a recovery rate of <40% were defined as: samples whose PRL determination results were interfered by macroprolactin (middle columns, accounting for y%);

- Samples with a recovery rate between 40% and 60% were defined as: samples that required further use of other means (e.g., gel filtration chromatography or clinical manifestations) to determine whether the PRL determination results were interfered by macroprolactin (right column, accounting for z%).
  where x + y + z = 100.

(2.4.2) Determination of samples prepared by gel filtration chromatography

For each sample (sample number = 1, 2, ..., n, where n = sample size), the measured PRL concentration values were connected by line segments to create line plots. The closeness between the line plot generated using each antibody kit for the initial samples and the line plot for the monomeric PRL samples obtained via gel filtration chromatography was compared. The closer the line plot from an antibody kit applied to the initial samples is to the line plot from the monomeric PRL samples (prepared by gel filtration chromatography), the higher the accuracy of that antibody kit in measuring monomeric PRL in the samples, indicating less interference from macroprolactin. Those skilled in the art will understand that for samples prepared by PEG precipitation, since macroprolactin interference has been removed, similar monomeric PRL concentrations would be expected when measured using different kits compared to the initial sample assessment.

(2.5) Results

(2.5.1) The products of the present disclosure vs. Commercial products

(2.5.1.1) Measurement results of samples prepared by PEG precipitation

As shown in FIG. 2A,

- among the above samples prepared by PEG precipitation and measured by present product 1, the proportion of samples whose PRL measurement results were not interfered by macroprolactin (up to 92.0%) was the highest, the proportion of samples whose PRL measurement results were interfered by macroprolactin (only 1.0%) and the proportion of samples that needed to be further determined by other means to determine whether the PRL measurement results were interfered by macroprolactin (only 7.0%) were the lowest;

- among the above samples prepared by PEG precipitation and measured by present product 2, the proportion of samples whose PRL measurement results were not interfered by macroprolactin (up to 88.0%) was the second highest, and the proportion of samples whose PRL measurement results were interfered by macroprolactin (only 5.0%) and the proportion of samples that needed to be further determined by other means to determine whether the PRL measurement results were interfered by macroprolactin (only 7.0%) were the second lowest;

- in comparison, among the above samples prepared by PEG precipitation and measured by commercial products 1, 2 and 3, the proportion of samples whose PRL measurement results were not interfered by macroprolactin (only 71.0%, 78.0% and 75.0%, respectively) were significantly lower, the proportion of samples whose PRL measurement results were interfered by macroprolactin (up to 18.0%, 12.0% and 13.0%, respectively) and the proportion of samples that needed to be further determined by other means to determine whether the PRL measurement results were interfered by macroprolactin (up to 11.0%, 10.0% and 12.0%, respectively) were significantly higher.

(2.5.1.2) Results of samples prepared by gel filtration chromatography

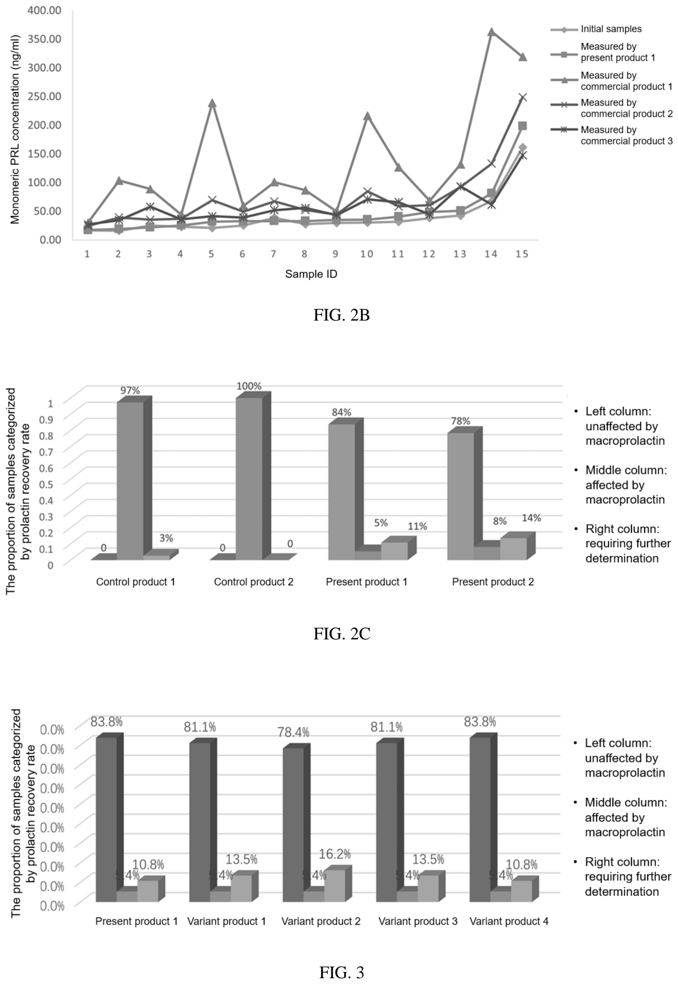

As shown in FIG. 2B,

- the concentration values of monomeric PRL in the samples prepared by gel filtration chromatography and measured by present product 1 were almost the same as the concentration values of PRL in the initial samples;

- in comparison, the concentration values of monomeric PRL in the samples prepared by gel filtration chromatography and measured by commercial products 1, 2 and 3 all deviated significantly to varying degrees from the concentration values of PRL in the initial samples.

(2.5.2) The products of the present disclosure vs. Control products

Among the samples prepared by PEG precipitation and measured by commercial product 1, the samples whose PRL determination results were interfered by macroprolactin were selected to compare the measurement performance of Present product 1 and Present product 2 against that of control products 1 and 2. The results are shown in FIG. 2C:

- among the samples prepared by PEG precipitation and measured by present product 1, the proportion of samples whose PRL determination results were not interfered by macroprolactin (up to 84%) was the highest, while the proportion of samples whose PRL determination results were interfered by macroprolactin (only 5%) and the proportion of samples that needed be further determined by other means to determine whether the PRL determination results were interfered by macroprolactin (only 11%) were the lowest;

- among the above samples prepared by PEG precipitation and measured by present product 2, the proportion of samples whose PRL measurement results were not interfered by macroprolactin (up to 78%) was the second highest, and the proportion of samples whose PRL measurement results were interfered by macroprolactin (only 8%) and the proportion of samples that needed to be further determined by other means to determine whether the PRL measurement results were interfered by macroprolactin (only 14%) were the second lowest;

- in comparison, the above samples prepared by PEG precipitation and measured by control products 1 and 2 were all samples whose PRL measurement results were interfered by macroprolactin (accounting for up to 97% and 100%, respectively) and samples that needed to be further determined by other means to determine whether the PRL measurement results were interfered by macroprolactin (accounting for 3% and 0%, respectively), and there was not a sample whose PRL measurement result was not interfered by macroprolactin (accounting for 0%).

(2.6) Conclusion

The results in FIGS. 2A and 2B above demonstrated that the current mainstream commercial products 1, 2, and 3 remain susceptible to interference from macroprolactin present in samples when measuring monomeric PRL concentration, meaning they readily misidentify macroprolactin as monomeric PRL. In contrast, the antibody kit comprising the antibody pair of the present disclosure (Present product 1 and Present product 2) are less susceptible to interference from macroprolactin during the measurement of monomeric PRL concentration in samples, showing a low tendency to misidentify macroprolactin as monomeric PRL. Consequently, they offer significant advantages for the specific determination of monomeric PRL in samples.

The above results of FIG. 2C showed that for the samples whose determination results of PRL were interfered by macroprolactin among the samples prepared by PEG precipitation and measured by commercial product 1,

- control products 1 and 2 that were the kits consisting of the anti-human mature PRL antibody pair capable of binding to the non-N-terminal and non-C-terminal regions, exhibited no ability to distinguish and resist interference;

- in contrast, the antibody kits of the present disclosure (present product 1 and present product 2) that were prepared with the antibody pairs consisting of an antibody capable of binding to the N-terminal region of human mature PRL (especially the region of aa1-13 of SEQ ID NO: 1 (SEQ ID NO: 2), more specifically the residues of aa1-3 and aa10-13) and an antibody capable of binding to the C-terminal region (especially the region of aa183-199 of SEQ ID NO: 1 (SEQ ID NO: 3), more specifically the residues of aa184-186 and aa199), exhibited good ability to distinguish and resist interference.

It is further demonstrated that the significant advantages of the antibody pair of the present disclosure in the specific determination of monomeric PRL in samples.

Example 3: Verification of effect of variant monoclonal antibodies

(3.1) Preparation of variant monoclonal antibodies

In addition to the "antibody 1a" and "antibody 2a" used in Examples 1 and 2, other variants of antibody 1 and antibody 2 (i.e., "antibodies 1b to 1p" and "antibodies 2b to 2h") were further prepared as shown in the following table:

| | | Antibody 1 | | Antibody 2 | |
|---|---|---|---|---|---|
| Heavy chain | CDR1 | GFIFX$_1$WYV | SEQ ID NO: 24 | GASITSDYA | SEQ ID NO: 16 |
| | CDR2 | ISGGX$_2$SHT | SEQ ID NO: 28 | ISFGGGHT | SEQ ID NO: 17 |
| | CDR3 | TRHSPYDHGDFGY | SEQ ID NO: 8 | ARHSPNDHGDTNF | SEQ ID NO: 18 |
| | CDR1 | QTLVHSLGNTY | SEQ ID NO: 11 | QDISX$_3$NG | SEQ ID NO: 47 |
| Light chain | CDR2 | KVSIA | SEQ ID NO: 12 | KTSX$_4$V | SEQ ID NO: 49 |
| | CDR3 | SQTTHVPTF | SEQ ID NO: 13 | QQGNTVPLT | SEQ ID NO: 23 |

wherein,
• $X_1$ was selected from: S, T, A or G, and the specific sequences of HCDR1 of antibody 1 were as follows:

| $X_1$ | Heavy chain CDR1 in antibody 1 | SEQ ID NO: 24 |
|---|---|---|
| S | GFIFSWYV | SEQ ID NO: 6 |
| T | GFIFTWYV | SEQ ID NO: 25 |
| A | GFIFAWYV | SEQ ID NO: 26 |
| G | GFIFGWYV | SEQ ID NO: 27 |

• $X_2$ was selected from: D, E, Q or N, and the specific sequences of HCDR2 of antibody 1 were as follows:

| $X_2$ | Heavy chain CDR2 in antibody 1 | SEQ ID NO: 28 |
|---|---|---|
| D | ISGGDSHT | SEQ ID NO: 7 |
| E | ISGGESHT | SEQ ID NO: 29 |
| Q | ISGGQSHT | SEQ ID NO: 30 |
| N | ISGGNSHT | SEQ ID NO: 31 |

• $X_3$ was selected from: D or E, and the specific sequences of LCDR1 of antibody 2 were as follows:

| $X_3$ | Light chain CDR1 in antibody 2 | SEQ ID NO: 47 |
|---|---|---|
| D | QDISDNG | SEQ ID NO: 21 |
| E | QDISENG | SEQ ID NO: 48 |

• $X_4$ was selected from: G, A, S or T, and the specific sequences of LCDR2 in antibody 2 were as follows:

| $X_4$ | Light chain CDR2 in antibody 2 | SEQ ID NO: 49 |
|---|---|---|
| G | KTSGV | SEQ ID NO: 22 |
| A | KTSAV | SEQ ID NO: 50 |
| S | KTSSV | SEQ ID NO: 51 |
| T | KTSTV | SEQ ID NO: 52 |

Therefore,
The specific sequences of the variable regions of antibody 1 were as follows:

| Antibody 1 | $X_1$ | $X_2$ | Heavy chain variable region | Light chain variable region |
|---|---|---|---|---|
| Antibody 1a | S | D | SEQ ID NO: 5 | SEQ ID NO: 10 |
| Antibody 1b | S | E | SEQ ID NO: 32 | SEQ ID NO: 10 |
| Antibody 1c | S | Q | SEQ ID NO: 33 | SEQ ID NO: 10 |
| Antibody 1d | S | N | SEQ ID NO: 34 | SEQ ID NO: 10 |
| Antibody 1e | T | D | SEQ ID NO: 35 | SEQ ID NO: 10 |
| Antibody 1f | T | E | SEQ ID NO: 36 | SEQ ID NO: 10 |
| Antibody 1g | T | Q | SEQ ID NO: 37 | SEQ ID NO: 10 |
| Antibody 1h | T | N | SEQ ID NO: 38 | SEQ ID NO: 10 |
| Antibody 1i | A | D | SEQ ID NO: 39 | SEQ ID NO: 10 |
| Antibody 1j | A | E | SEQ ID NO: 40 | SEQ ID NO: 10 |
| Antibody 1k | A | Q | SEQ ID NO: 41 | SEQ ID NO: 10 |
| Antibody 1l | A | N | SEQ ID NO: 42 | SEQ ID NO: 10 |
| Antibody 1m | G | D | SEQ ID NO: 43 | SEQ ID NO: 10 |
| Antibody 1n | G | E | SEQ ID NO: 44 | SEQ ID NO: 10 |
| Antibody 1o | G | Q | SEQ ID NO: 45 | SEQ ID NO: 10 |
| Antibody 1p | G | N | SEQ ID NO: 46 | SEQ ID NO: 10 |

The specific sequences of the variable regions of antibody 2 were as follows:

| Antibody 2 | $X_3$ | $X_4$ | Heavy chain variable region | Light chain variable region |
|---|---|---|---|---|
| Antibody 2a | D | G | SEQ ID NO: 15 | SEQ ID NO: 20 |
| Antibody 2b | D | A | SEQ ID NO: 15 | SEQ ID NO: 53 |
| Antibody 2c | D | S | SEQ ID NO: 15 | SEQ ID NO: 54 |
| Antibody 2d | D | T | SEQ ID NO: 15 | SEQ ID NO: 55 |
| Antibody 2e | E | G | SEQ ID NO: 15 | SEQ ID NO: 56 |
| Antibody 2f | E | A | SEQ ID NO: 15 | SEQ ID NO: 57 |
| Antibody 2g | E | S | SEQ ID NO: 15 | SEQ ID NO: 58 |
| Antibody 2h | E | T | SEQ ID NO: 15 | SEQ ID NO: 59 |

(3.2) Preparation of variant antibody kits

The variant monoclonal antibodies prepared in (3.1) were used to prepare the antibody kits shown in the following table.

| Antibody kit | Capture reagent | | Detection reagent | |
|---|---|---|---|---|
| | Capture antibody | Magnetic beads | Detection antibody | Label |
| Variant product 1 | Antibody 1c | Superparamagnetic microbeads | Antibody 2a | Alkaline phosphatase |
| Variant product 2 | Antibody 1i | | Antibody 2c | |
| Variant product 3 | Antibody 1k | | Antibody 2f | |
| Variant product 4 | Antibody 1a | | Antibody 2c | |

In the above antibody kits,

- the above capture antibody was coated on superparamagnetic microbeads to serve as a capture reagent; and

- the above detection antibody was labeled with alkaline phosphatase to serve as a detection reagent.

(3.3) Determination of monomeric PRL in samples

The samples whose PRL determination results were interfered by macroprolactin" among the samples prepared by PEG precipitation and measured by commercial product 1 in Example 2 were used as the test samples.
Using variant products 1 to 4, the above test samples were measured as described in Example 2, and the results were compared with the results obtained by determining the above test samples using present product 1.

(3.4) Results

As shown in FIG. 3, similar to the determination results obtained by using present product 1, among the above samples prepared by PEG precipitation and measured by variant products 1 to 4,

- the proportion of samples whose PRL determination results were not interfered by macroprolactin was also high (81.1%, 78.4%, 81.1%, and 83.8%, respectively);

- the proportion of samples whose PRL determination results were interfered by macroprolactin was also low (only 5.4%, 5.4%, 5.4%, and 5.4%, respectively);

- the proportion of samples that needed to be further determined by other means to determine whether the PRL determination results were interfered by macroprolactin was also low (only 13.5%, 16.2%, 13.5%, and 10.8%, respectively).

(3.5) Conclusion

The antibody kits (variant products 1 to 4) prepared using antibody pairs consisting of conservative variants of an antibody capable of binding to the N-terminal region of human mature PRL (particularly the region of aa1-13 of SEQ ID NO: 1 (SEQ ID NO: 2), more specifically the residues of aa1-3 and aa10-13) and conservative variants of an antibody capable of binding to the C-terminal region (particularly the region of aa183-199 of SEQ ID NO: 1 (SEQ ID NO: 3), more specifically the residues of aa184-186 and aa199) could also achieve the same/similar effects as those achieved when using present product 1.

**Claims**

**1.** An antibody pair capable of binding to human mature prolactin, wherein

the antibody pair comprises:

a first monoclonal antibody or antigen-binding fragment thereof, which is capable of binding to the N-terminal region of human mature prolactin, and

a second monoclonal antibody or antigen-binding fragment thereof, which is capable of binding to the C-terminal region of human mature prolactin, and

the human mature prolactin consists of the amino acid sequence as set forth in SEQ ID NO: 1.

2. The antibody pair according to claim 1, wherein

the first monoclonal antibody or antigen-binding fragment thereof is capable of binding to a region within amino acids 1 to 13 of SEQ ID NO: 1, and/or

the second monoclonal antibody or antigen-binding fragment thereof is capable of binding to a region within amino acids 183 to 199 of SEQ ID NO: 1.

3. The antibody pair according to claim 2, wherein

the region within amino acids 1 to 13 of SEQ ID NO: 1 comprises at least amino acids 1 to 3 and 10 to 13, and optionally comprises one or more conservative amino acid insertions, deletions or substitutions in the portion other than amino acids 1 to 3 and 10 to 13, and/or

the region within amino acids 183 to 199 of SEQ ID NO: 1 comprises at least amino acids 184 to 186 and 199, and optionally comprises one or more conservative amino acid insertions, deletions or substitutions in the portion other than amino acids 184 to 186 and 199.

4. The antibody pair according to claim 1, wherein

the first monoclonal antibody or antigen-binding fragment thereof comprises:

a heavy chain variable region, which comprises:

CDR1 comprising the amino acid sequence of $GFTFX_1WYV$,
CDR2 comprising the amino acid sequence of $ISGGX_2SHT$, and
CDR3 comprising the amino acid sequence of TRHSPYDHGDFGY, and

a light chain variable region, which comprises:

CDR1 comprising the amino acid sequence of QTLVHSLGNTY,
CDR2 comprising the amino acid sequence of KVSIA, and
CDR3 comprising the amino acid sequence of SQTTHVPTF; and/or

the second monoclonal antibody or antigen-binding fragment thereof comprises:

a heavy chain variable region, which comprises:

CDR1 comprising the amino acid sequence of GASITSDYA,
CDR2 comprising the amino acid sequence of ISFGGGHT, and
CDR3 comprising the amino acid sequence of ARHSPNDHGDTNF, and

a light chain variable region, which comprises:

CDR1 comprising the amino acid sequence of $QDISX_3NG$,
CDR2 comprising the amino acid sequence of $KTSX_4V$, and
CDR3 comprising the amino acid sequence of QQGNTVPLT,

wherein,

$X_1$ is selected from the group consisting of: S, T, A or G;
$X_2$ is selected from the group consisting of: D, E, Q or N;
$X_3$ is selected from the group consisting of: D or E; and/or

$X_4$ is selected from the group consisting of: G, A, S or T;

preferably wherein:

$X_1$ is S or A;
$X_2$ is D or Q;
$X_3$ is D or E; and/or
$X_4$ is G, A or S.

5. The antibody pair according to claim 4, wherein

the first monoclonal antibody or antigen-binding fragment thereof comprises:

a heavy chain variable region, which comprises an amino acid sequence selected from SEQ ID NOs: 5 and 32 to 46, and
a light chain variable region, which comprises an amino acid sequence as set forth in SEQ ID NO: 10; and/or

the second monoclonal antibody or antigen-binding fragment thereof comprises:

a heavy chain variable region, which comprises an amino acid sequence as set forth in SEQ ID NO: 15, and
a light chain variable region, which comprises an amino acid sequence selected from SEQ ID NOs: 20 and 53 to 59.

6. A monoclonal antibody or antigen-binding fragment thereof capable of binding to the N-terminal region of human mature prolactin, which comprises:

a heavy chain variable region, which comprises:

CDR1, which comprises the amino acid sequence of GFIF$X_1$WYV,
CDR2, which comprises the amino acid sequence of ISGG$X_2$SHT, and
CDR3, which comprises the amino acid sequence of TRHSPYDHGDFGY, and

a light chain variable region, which comprises:

CDR1, which comprises the amino acid sequence of QTLVHSLGNTY,
CDR2, which comprises the amino acid sequence of KVSIA, and
CDR3, which comprises the amino acid sequence of SQTTHVPTF,

wherein,

$X_1$ is selected from the group consisting of: S, T, A or G;
$X_2$ is selected from the group consisting of: D, E, Q or N;

preferably wherein:

$X_1$ is S or A; and
$X_2$ is D or Q.

7. The monoclonal antibody or antigen-binding fragment thereof according to claim 6, which comprises:

a heavy chain variable region, which comprises an amino acid sequence selected from SEQ ID NOs: 5 and 32 to 46, and
a light chain variable region, which comprises an amino acid sequence as set forth in SEQ ID NO: 10.

8. A monoclonal antibody or antigen-binding fragment thereof capable of binding to the C-terminal region of human mature prolactin, which comprises:

a heavy chain variable region, which comprises:

CDR1, which comprises the amino acid sequence of GASITSDYA,
CDR2, which comprises the amino acid sequence of ISFGGGHT, and
CDR3, which comprises the amino acid sequence of ARHSPNDHGDTNF, and

a light chain variable region, which comprises:

CDR1, which comprises the amino acid sequence of QDISX$_3$NG,
CDR2, which comprises the amino acid sequence of KTSX$_4$V, and
CDR3, which comprises the amino acid sequence of QQGNTVPLT,

wherein,

X$_3$ is selected from the group consisting of: D or E; and
X$_4$ is selected from the group consisting of: G, A, S or T;

preferably wherein:

X$_3$ is D or E; and
X$_4$ is G, A or S.

9.  The monoclonal antibody or antigen-binding fragment thereof according to claim 8, which comprises:

a heavy chain variable region, which comprises an amino acid sequence as set forth in SEQ ID NO: 15, and
a light chain variable region, which comprises an amino acid sequence selected from SEQ ID NOs: 20 and 53 to 59.

10. A method for determining prolactin in a biological sample, comprising:

(a) contacting a biological sample suspected of containing prolactin with the antibody pair according to any one of claims 1 to 5, and
(b) quantifying the prolactin in the biological sample according to a signal generated in step (a).

11. The method according to claim 10, wherein the prolactin in the biological sample is quantified according to a chemiluminescent signal generated in step (a).

12. The method according to claim 10 or 11, wherein the determination is independent of isolating monomeric mature prolactin from the biological sample.

13. The method according to claim 10 or 11, wherein

(1) before step (a), the biological sample is pre-divided into a first part and a second part:

(1A) the first part is not subjected to any treatment and serves as a first test sample; and
(1B) the second part is mixed with an equal volume of a solution containing polyethylene glycol, followed by vortexing and centrifugation to obtain a supernatant, which serves as a second test sample;

(2) determining the prolactin concentrations in the first test sample and the second test sample through steps (a) and (b) as a first concentration and a second concentration, respectively; and
(3) calculating the recovery rate using the following formula:

Recovery rate of prolactin (%) = (the second concentration $\times$ 2)/(the first concentration) $\times$ 100;

wherein the recovery rate is greater than 60%, preferably greater than 80%, and more preferably greater than 90%.

14. A method for producing a monoclonal antibody, which comprises immunizing an animal with the following peptide:

the N-terminal region of human mature prolactin, and/or
the C-terminal region of human mature prolactin,
the human mature prolactin consists of the amino acid sequence as set forth in SEQ ID NO: 1.

**15.** The method according to claim 14, wherein

the N-terminal region of human mature prolactin is a region within amino acids 1 to 13 of SEQ ID NO: 1, and/or
the C-terminal region of human mature prolactin is a region within amino acids 183 to 199 of SEQ ID NO: 1;
preferably wherein

the region within amino acids 1 to 13 of SEQ ID NO: 1 comprises at least amino acids 1 to 3 and 10 to 13, and
optionally comprises one or more conservative amino acid insertions, deletions or substitutions in the part
other than amino acids 1 to 3 and 10 to 13, and/or
the region within amino acids 183 to 199 of SEQ ID NO: 1 comprises at least amino acids 184 to 186 and 199,
and optionally comprises one or more conservative amino acid insertions, deletions or substitutions in the
part other than amino acids 184 to 186 and 199.

FIG. 1A

FIG. 1B

FIG. 2A

FIG. 2B

FIG. 2C

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202411798937